# EUROPÄISCHE PATENTSCHRIFT

(11) **EP 3 428 610 B1**
(45) Veröffentlichungstag und Bekanntmachung des Hinweises auf die Patenterteilung: **29.01.2020**
(21) Anmeldenummer: 18177053.8
(22) Anmeldetag: 11.06.2018
(51) Int. Cl.: G01N 1/20, G01N 33/02, C12M 1/12, C12M 1/26

(54) **PROBENAHMEVORRICHTUNG**
SAMPLING DEVICE
DISPOSITIF D'ÉCHANTILLONNAGE

(30) Priorität: 06.07.2017 AT 505632017
(43) Veröffentlichungstag der Anmeldung: 16.01.2019
(73) Patentinhaber: Zeta Biopharma GmbH, 8501 Lieboch (AT)
(72) Erfinder: Trummer, Erwin, 8083 St. Stefan im Rosental (AT)
(74) Vertreter: Röggla, Harald

(56) Entgegenhaltungen:
- WO-A1-2004/025271
- DE-B3-102005 022 966
- DE-B3-102015 109 080
- US-A1- 2002 041 830

## Beschreibung

Die Erfindung betrifft eine Probenahmevorrichtung zur Befüllung eines Probengefäßes mit Probenmaterial aus einem Materialbehälter.

Des Weiteren betrifft die Erfindung ein Verfahren zur Befüllung eines Probengefäßes mit Probenmaterial aus einem Materialbehälter mit einer Probenahmevorrichtung.

In den Bereichen der Biopharmazie und der Lebensmittelindustrie werden hohe Standards in Bezug auf Produktion, Lagerung und Transport von Produkten angelegt. Gesetzliche Vorgaben in diesen Bereichen regulieren unter anderem die Sterilität von Produktionsprozessen, Verarbeitungsprozessen und Anwendungen der Produkte sowie die Durchführung von Produktprüfungen. Im Rahmen der Produktion besteht somit aufgrund von Qualitätssicherungsanforderungen und den gesetzlichen Vorgaben die Notwendigkeit Produkte laufend auf Kontaminationen durch Keime und Bakterien zu überprüfen. Hierdurch wird gewährleistet, dass die Qualität der Produktion den gesetzlichen Standards entspricht.

In der Regel befinden sich Produkte der Biopharmazie oder der Lebensmittelindustrie im Produktionsprozess in großen aseptischen Materialbehältern in welchen diese transportiert, zwischengelagert oder verarbeitet werden. Gemäß dem Stand der Technik erfolgen Probenahmen aus den Materialbehältern indem diese geöffnet, ein Teil des Produkts aus dem Materialbehälter mit einer möglichst sterilen Vorrichtung entnommen, und in ein zuvor sterilisiertes Probengefäß gefüllt wird. Insbesondere bei der Beprobung von hochstückigen Materialien, wie beispielsweise Fruchtzubereitungen mit großen Fruchtstücken in der Lebensmittelindustrie, besteht die Notwendigkeit den Materialbehälter zu öffnen, da die Fruchtstücke schwer aus dem Materialbehälter abgesaugt werden können. Diese Form der Probenahme entspricht den gesetzlichen Vorgaben, und erlaubt die Validierung von Produktchargen, welche keine Kontamination aufweisen.

Probenahmevorrichtungen gemäß dem Stand der Technik, welche auf diesem Prinzip beruhen, weisen jedoch den Nachteil auf, dass bei der Entnahme von Probenmaterial der aseptische Materialbehälter geöffnet werden muss. Hierdurch entsteht ein erhebliches Risiko das Probenmaterial bei der Probenahme zu kontaminieren. Dies kann dazu führen, dass die Probe unbemerkt kontaminiert wird, obwohl das Produkt im Materialbehälter selbst keine Kontamination aufweist. Infolge eines positiven Tests auf Kontamination des Probenmaterials wird daraufhin das eigentlich kontaminationsfreie Produkt im Materialbehälter verworfen. Dies führt zu einem erheblichen wirtschaftlichen Schaden für den Produzenten, da in so einem Materialbehälter mehrere Tonnen des Produkts enthalten sein können. Zusätzlich besteht das Risiko das Produkt im Materialbehälter selbst im Zuge der Probenahme unbemerkt zu kontaminieren, was den Nachteil einer Kontaminationsverschleppung in der Produktionsanlage zur Folge haben kann.

Das Dokument DE 10 2005 022 966 B3 offenbart eine aseptische Probenahmestation mit zur Befüllung eines Probengefäßes mit Probenmaterial aus einem Materialbehälter.

Das Dokument WO 2004/025271 A1 offenbart eine Injektionsnadel und ein Verfahren zum Transfer einer Fluidprobe in ein Probengefäß.

Das Dokument US 2002/0041830 A1 offenbart ein System zur multiplen Probennahme und Probenisolierung.

Es ist die Aufgabe der vorliegenden Erfindung eine Probenahmevorrichtung und ein Verfahren zu schaffen, welche die oben angeführten Nachteile vermeiden.

Erfindungsgemäß wird die vorliegende Aufgabe dadurch gelöst, dass die Probenahmevorrichtung eine erste Absperreinheit, eine Sterilisationseinheit und eine Entnahmekammer umfasst, wobei die Entnahmekammer einen Kammerinnenraum, eine Manipulationseinheit, eine in dem Kammerinnenraum angeordnete Öffnungseinheit sowie eine in dem Kammerinnenraum angeordnete Kopplungseinheit aufweist, die erste Absperreinheit zwischen dem Materialbehälter und der Kopplungseinheit angeordnet ist, und die Sterilisationseinheit mit dem Kammerinnenraum verbunden und dazu ausgebildet ist, den Kammerinnenraum zu sterilisieren, wobei die Manipulationseinheit dazu ausgebildet ist das Probengefäß zumindest teilweise in den Kammerinnenraum, beweglich zwischen der Öffnungseinheit und der Kopplungseinheit, aufzunehmen, die Öffnungseinheit dazu ausgebildet ist das Probengefäß zu öffnen und zu schließen, und die Kopplungseinheit mit dem Probengefäß koppelbar ausgebildet ist.

Die vorliegende Aufgabe wird außerdem durch ein Verfahren zur Befüllung eines Probengefäßes mit Probenmaterial aus einem Materialbehälter mit einer Probenahmevorrichtung mit folgenden Verfahrensschritten gelöst:
a) Fluten eines Kammerinnenraums der Probenahmevorrichtung mit Heißdampf einer Temperatur von zumindest 120 °C;
b) Reduzieren der Temperatur des Heißdampfes auf unter 120 °C;
c) Einführen des Probengefäßes in den Kammerinnenraum;
d) Öffnen des Probengefäßes;
e) Verbinden des Probengefäßes mit dem Materialbehälter;
f) Befüllen des Probengefäßes mit Probenmaterial aus dem Materialbehälter;
g) Schließen des Probengefäßes vorzugsweise unter Heißdampf;
h) Entfernen des Probengefäßes aus dem Kammerinnenraum.

Die erfindungsgemäße Ausführung der Probenahmevorrichtung sieht eine erste Absperreinheit, eine Sterilisationseinheit und eine Entnahmekammer vor. Die Entnahmekammer selbst weist eine Manipulationseinheit und einen Kammerinnenraum auf, in welchen für die Durchführung einer aseptischen Probenahme ein geschlossenes und sterilisiertes Probengefäß eingebracht wird. Im Kammerinnenraum befinden sich zudem eine Öffnungseinheit und eine Kopplungseinheit, welche mit der ersten Absperreinheit verbunden ist. Die erste Absperreinheit ist mit dem Materialbehälter verbunden und kann beispielsweise in Form eines Ventils vorliegen. Die Sterilisationseinheit, welche beispielsweise in Form eines Dampferzeugers vorliegen kann, ist mit dem Kammerinnenraum verbunden und sterilisiert im Zuge der Probenahme den Kammerinnenraum und die weiteren Bestandteile der Probenahmevorrichtung welche sich im Kammerinnenraum befinden. Das Probengefäß wird mittels der Öffnungseinheit geöffnet, mittels der Manipulationseinheit von der Öffnungseinheit zur Kopplungseinheit bewegt, und anschließend mit der Kopplungseinheit verbunden. Danach wird die erste Absperreinheit geöffnet, und eine definierte Menge an Probenmaterial aus dem Probenbehälter in das Probengefäß gefüllt. Im Anschluss wird das Probengefäß von der Kopplungseinheit getrennt und mittels der Manipulationseinheit erneut zur Öffnungseinheit bewegt. Mittels der Öffnungseinheit wird das Probengefäß im Anschluss wieder geschlossen und danach wird das Probengefäß aus der Entnahmekammer entnommen.

Durch die erfindungsgemäße Ausführung der Probenahmevorrichtung wird der Vorteil erreicht, dass eine vollkommen aseptische und abgeschlossene Umgebung für die Probenahme bereitgestellt wird. Besonders vorteilhaft ist hierbei, dass vor der Öffnung der ersten Absperreinheit der Kammerinnenraum der Entnahmekammer durch die Sterilisationseinheit sterilisiert wird, wodurch etwaige Kontaminationen, welche mit dem Probengefäß in den Kammerinnenraum eingebracht wurden, entfernt werden. Dies ermöglicht eine völlig aseptische Probenahme und verhindert mögliche Kontaminationen des Probenmaterials oder des Materialbehälters.

Besonders vorteilhaft ist, dass der Prozess der Sterilisation durch die Sterilisationseinheit abgeschlossen im Kammerinnenraum stattfindet. Dies minimiert das Verletzungsrisiko für Arbeitskräfte welches in der Regel mit der Anwendung von Heißdampf oder Chemikalien für die Sterilisation verbunden ist.

Vorteilhafte Ausgestaltungen der erfindungsgemäßen Probenahmevorrichtung und des Verfahrens, sowie alternativer Ausführungsvarianten werden in weiterer Folge anhand der Figuren näher erläutert.

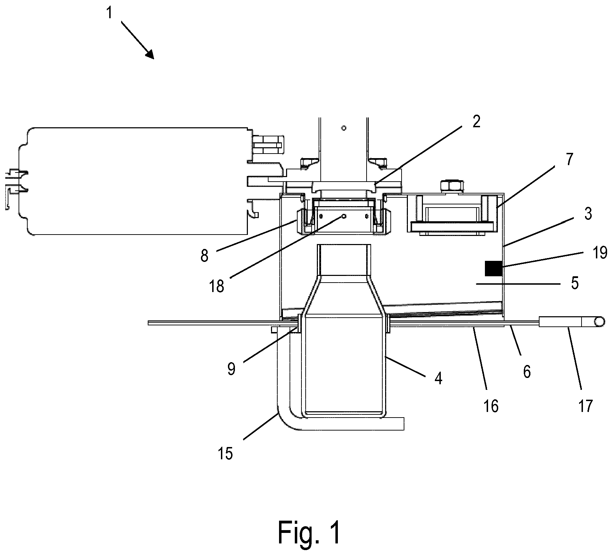

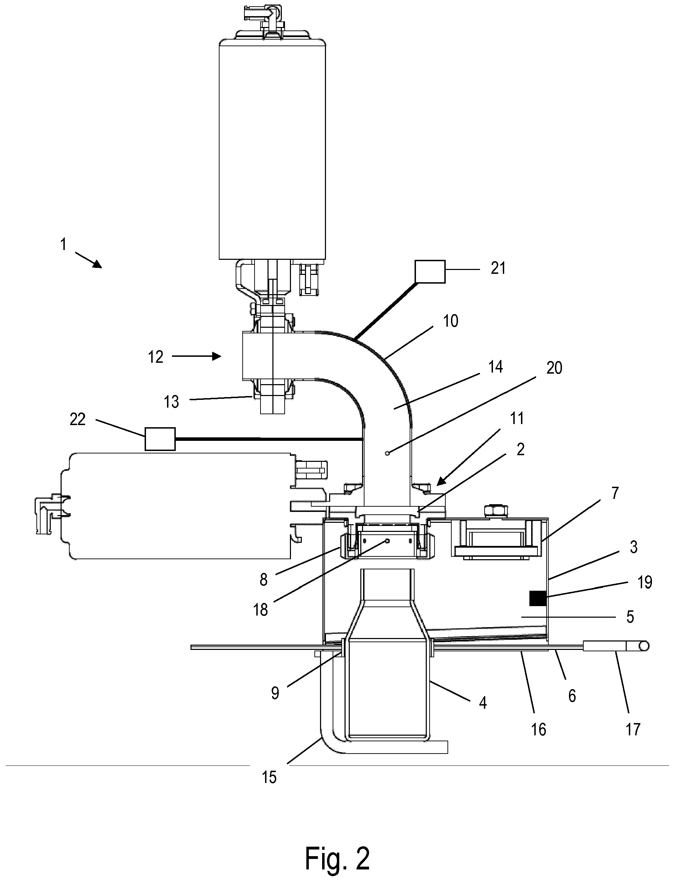

Figur 1 zeigt einen Schnitt durch eine erfindungsgemäße Probenahmevorrichtung mit einem teilweise in der Probenahmevorrichtung aufgenommenen Probengefäß in Seitenansicht. Figur 2 zeigt einen Schnitt durch eine erfindungsgemäße Probenahmevorrichtung mit einem teilweise in der Probenahmevorrichtung aufgenommenen Probengefäß und einer Entnahmeleitung in Seitenansicht.

Figur 1 zeigt eine Schnittansicht einer erfindungsgemäßen Probenahmevorrichtung 1, mit einer ersten Absperreinheit 2, einer Sterilisationseinheit, welche in Figur 1 nicht dargestellt ist, und einer Entnahmekammer 3. In der in Figur 1 dargestellten Probenahmevorrichtung 1 ist ein Probengefäß 4 teilweise in der Entnahmekammer 3 aufgenommen. Die Entnahmekammer 3 weist einen Kammerinnenraum 5, eine Manipulationseinheit 6, eine in dem Kammerinnenraum 5 angeordnete Öffnungseinheit 7 sowie eine Kopplungseinheit 8 auf, welche sich ebenfalls innerhalb des Kammerinnenraums 5 befindet. Die erste Absperreinheit 2 ist zwischen einem Materialbehälter, welcher in Figur 1 nicht dargestellt ist, und der Kopplungseinheit 8 angeordnet, und ermöglicht beziehungsweise verhindert einen Durchfluss von Probenmaterial aus dem Materialbehälter in das Probengefäß 4. Die Öffnungseinheit 7 und die Kopplungseinheit 8 sind in der in Figur 1 dargestellten Ausführungsform der Probenahmevorrichtung 1 nebeneinander angeordnet. Die erste Absperreinheit 2 ist beispielsweise als elektrisch, pneumatisch oder mechanisch betätigtes Ventil ausgeführt. Weitere Ausführungsvarianten der ersten Absperreinheit 2 ergeben sich für den Fachmann aus diesem beispielhaften Verweis.

Die Sterilisationseinheit ist dazu ausgebildet den Kammerinnenraum 5 zu sterilisieren, wobei die Sterilisationseinheit mit dem Kammerinnenraum 5 verbunden ist. Bei diesem Vorgang werden auch alle weiteren sich darin befindlichen Elemente sterilisiert. In der bevorzugten Ausführungsvariante der Probenahmevorrichtung 1 ist die Sterilisationseinheit als Dampferzeuger ausgeführt, welcher im Zuge eines Sterilisationsvorganges den Kammerinnenraum 5 mit Heißdampf flutet. Dies bietet den Vorteil, dass Dampferzeuger am Markt weit verbreitet und somit einfach verfügbar sind. Zudem benötigen diese keine potentiell gesundheitsschädlichen Chemikalien für den Sterilisationsvorgang. Besonders bevorzugt ist, dass der Dampferzeugter Dampf einer Temperatur von zumindest 120°C erzeugt. Dies bietet den Vorteil, dass hierdurch ein besonders gutes Ergebnis des Sterilisationsvorganges erzielt wird. In einer alternativen Ausführungsvariante sterilisiert die Sterilisationseinheit den Kammerinnenraum 5 beispielsweise mit chemischen Methoden. Weitere Ausführungsvarianten der Sterilisationseinheit ergeben sich für den Fachmann aus diesem beispielhaften Verweis.

In einer bevorzugten Ausführungsvariante weist die Probenahmevorrichtung 1 einen ersten Temperatursensor 19 auf, welcher im Kammerinnenraum 5 angeordnet ist. Dies bietet den Vorteil, dass hierdurch ermöglich wird, die Dampftemperatur im Kammerinnenraum 5 zu bestimmen, und somit zu verhindern dass diese unter einen bestimmten vorgegebenen Wert fällt. Besonders vorteilhaft ist, dass durch diese Überprüfung gewährleistet wird, dass der Sterilisationsprozess bei jeder Probenahme besonders effektiv erfolgt.

Die Manipulationseinheit 6 nimmt das Probengefäß 4 zumindest teilweise in den Kammerinnenraum 5, beweglich zwischen der Öffnungseinheit 7 und der Kopplungseinheit 8 auf. Die Manipulationseinheit 6 ist in der in Figur 1 dargestellten bevorzugten Ausführungsvariante der Manipulationseinheit 6 als eine verschiebbare Begrenzungsfläche der Entnahmekammer 3 ausgebildet, welche eine, einem Querschnitt des Probengefäßes 4 angepasste, Öffnung 9 aufweist. Die Manipulationseinheit 6 ist beispielsweise als ein verschiebbares Blech ausgeführt, welches die Begrenzungsfläche der Entnahmekammer 3 bildet. Zur Aufnahme des Probengefäßes 4 in die Entnahmekammer 3 weist die Manipulationseinheit 6 die dem Querschnitt des Probengefäßes 4 angepasste Öffnung 9 auf, in welche das Probengefäß 4 eingeführt wird. Bei einer Flutung des Kammerinnenraums 5 mit Heißdampf durch die Sterilisationseinheit dichtet das Probengefäß 4 die Öffnung 9 der Manipulationseinheit 6, und somit den Kammerinnenraum 5 ab. Hierdurch wird der Dampf im Kammerinnenraum 5 zurückgehalten, wodurch der Vorteil erreicht wird, dass keine besonderen Vorsichtsmaßnahmen im Umgang mit der erfindungsgemäßen Probenahmevorrichtung 1 erforderlich sind.

Das Probengefäß 4 wird im Zuge einer Probenahme mit der erfindungsgemäßen Probenahmevorrichtung 1 in die Öffnung 9 der Manipulationseinheit 6 eingeführt. Diese ist derart positioniert, dass das Probengefäß 4 mit der Öffnungseinheit 7 verbindbar ist. Das Probengefäß 4 selbst kann beispielsweise eine Laborflasche sein, und weist einen Verschluss auf. Die Öffnungseinheit 7 ist dazu ausgebildet das Probengefäß 4 zu öffnen und zu schließen. Die Öffnungseinheit 7 greift im Zuge dessen in den Verschluss des Probengefäßes 4 ein, und hält diesen nach dem Öffnen des Probengefäßes 4 an der Öffnungseinheit 7 zurück. Im Anschluss wird das Probengefäß 4 mittels der Manipulationseinheit 6 an die Position der Kopplungseinheit 8 bewegt. Die Kopplungseinheit 8 ist mit dem Probengefäß 4 koppelbar ausgebildet, wobei das geöffnete Probengefäß 4 beispielsweise in die Kopplungseinheit 8 eingeschraubt wird. In alternativen Ausführungsvarianten sind weitere Kopplungsmethoden wie beispielsweise Bajonettverschlüsse oder Klemmbefestigungen vorsehbar. Hierbei wird das Probengefäß 4 mit dem Materialbehälter verbunden. Im Anschluss an die Koppelung des Probengefäßes 4 an die Kopplungseinheit 8 wird die erste Absperreinheit 2 geöffnet und eine Menge an Probenmaterial aus dem Materialbehälter in das Probengefäß 4 gefüllt. Im Anschluss wird die erste Absperreinheit 2 wieder geschlossen und die Kopplung des Probengefäßes 4 mit der Kopplungseinheit 8 gelöst. In weiterer Folge wird das Probengefäß 4 mittels der Manipulationseinheit 6 wieder an die Position der Öffnungseinheit 7 verschoben, und durch die Öffnungseinheit 7, mittels des zuvor an der Öffnungseinheit 7 verbliebenen Verschlusses, verschlossen. Zuletzt wird das Probengefäß 4 aus der Manipulationseinheit 6 und somit aus dem Kammerinnenraum 5 entfernt. Der gesamte beschriebene Vorgang der Probenahme findet unter sterilen Bedingungen statt. Hierzu erzeugt in der bevorzugten Ausführungsvariante die Sterilisationseinheit eine Heißdampfatmosphäre im Kammerinnenraum 5. Das Öffnen des Probengefäßes 4, das Koppeln mit der Kopplungseinheit 8, der Befüllvorgang und das Verschließen des Probengefäßes 4 finden in der Heißdampfatmosphäre statt. Hierdurch ergibt sich der Vorteil, dass vorhandene Keime und Bakterien abgetötet werden und das Probenmaterial keimfrei bleibt. Darüber hinaus besteht durch diesen Vorgang kein Risiko den Inhalt des Materialbehälters zu kontaminieren.

In einer bevorzugten Ausführungsvariante der Kopplungseinheit 8 weist die Kopplungseinheit 8 Sterilisationsöffnungen 18 auf, welche mit der Sterilisationseinheit verbunden sind. Dies bietet den Vorteil, dass das Probengefäß 4 in jenem Bereich in welchen die Kopplungseinheit 8 eingreift im Zuge der Kopplung zusätzlich bedampft werden kann. Hierdurch ergibt sich der Vorteil, dass eventuell vorhandene Kontaminationen in diesem Bereich entfernt werden.

Die Entnahmekammer 3 weist in einer bevorzugen Ausführungsvariante Verbindungselemente 16 auf, wobei die Manipulationseinheit 6 in den Verbindungselementen 16 verschiebbar aufgenommen ist. Bei der wie zuvor beschriebenen Ausführung der Manipulationseinheit 6 als Blech sind die Verbindungselemente 16 beispielsweise als an der Entnahmekammer 3 angeordnete Führungsnuten ausgeführt, in welchen die Manipulationseinheit 6 zur Bewegung des Probengefäßes 4 verschoben werden kann. Dies bietet den Vorteil, dass die Verbindungselemente 16 eine sichere Führung für die Manipulationseinheit 6 bereitstellen, wodurch das Risiko minimiert wird, dass während des Sterilisationsvorganges Dampf aus dem Kammerinnenraum 5 austritt.

In einer besonders bevorzugten Ausführungsvariante weist die Manipulationseinheit 6 ein Griffstück 17 auf. Dies bietet den Vorteil, dass das Griffstück 17 eine leichte Handhabbarkeit der Manipulationseinheit 6 gewährleistet. Des Weiteren bietet das Griffstück 17 den Vorteil, dass die Manipulationseinheit 6 auch ohne zusätzliche Sicherheitsausrüstung bewegt werden kann, sollte sich die Manipulationseinheit 6 durch den Sterilisationsvorgang mit Heißdampf erwärmen.

Die erste Absperreinheit 2 der Probenahmevorrichtung 1 weist in einer bevorzugten Ausführungsvariante einen Durchgangsquerschnitt von zumindest 30 mm auf. Die Kopplungseinheit 8 ist des Weiteren dazu ausgebildet ein Probengefäß 4, welches einen Öffnungsbereich mit einem Durchmesser von zumindest 30 mm aufweist anzukoppeln. Dies bietet den Vorteil, dass die Probenahmevorrichtung 1 auch für inhomogene Materialien wie beispielsweise Fruchtzubereitungen, welche ganze Fruchtstücke beinhalten, einsetzbar ist.

Figur 2 zeigt einen Schnitt durch eine erfindungsgemäße Probenahmevorrichtung 1 in Seitenansicht in der bevorzugten Ausführungsvariante mit einer zusätzlichen Entnahmeleitung 10, welche einen Leitungsinnenraum 14 aufweist. Die Entnahmeleitung 10 weist des Weiteren ein erstes Ende 11, an welchem die erste Absperreinheit 2 angeordnet ist, und ein zweites Ende 12, an welchem eine zweite Absperreinheit 13 angeordnet ist auf. Das zweite Ende 12 der Entnahmeleitung 10 ist mit dem Materialbehälter verbunden, welcher in Figur 2 nicht dargestellt ist. Die erste Absperreinheit 2 und die zweite Absperreinheit 13 sind dazu ausgebildet, die Entnahmeleitung 10 abzusperren. Die erste Absperreinheit 2 und die zweite Absperreinheit 13 können unter anderem als elektrisch, pneumatisch oder mechanisch betätigte Ventile ausgeführt sein. Weitere Ausführungsvarianten der ersten Absperreinheit 2 und der zweiten Absperreinheit 13 ergeben sich für den Fachmann aus diesem beispielhaften Verweis.

Die Anordnung der Entnahmeleitung 10, welche durch die erste Absperreinheit 2 und die zweite Absperreinheit 13 am ersten Ende 11 und am zweiten Ende 12 absperrbar ist, zwischen dem Materialbehälter und der Entnahmekammer 3, bietet den Vorteil, dass die Entnahmeleitung 10 im Zuge der Probenahme mit einer definierten Menge an Probenmaterial befüllt wird. Hierzu weist der Leitungsinnenraum 14 ein Volumen auf, welches ein Volumen von Probenmaterial zur Befüllung des Probengefäßes 4 bestimmt. Dies bietet den Vorteil, dass die aus dem Materialbehälter entnommene Menge an Probenmaterial bei jeder Probenahme identisch ist. Im Zuge einer Probenahme wird zuerst die zweite Absperreinheit 13 geöffnet, und die erste Absperreinheit 2 geschlossen gehalten. Die Entnahmeleitung 10 füllt sich infolgedessen mit dem Probenmaterial aus dem Materialbehälter. Im Anschluss wir die zweite Absperreinheit 13 geschlossen und die erste Absperreinheit 2 geöffnet um das Probenmaterial in den Probenbehälter 4 zu füllen. Diese Ausführung der Entnahmeleitung 10 bietet zusätzlich den Vorteil, dass durch die sequentielle Öffnung der zweiten Absperreinheit 13 und der ersten Absperreinheit 2 eine zusätzliche Sicherheitsbarriere eingeführt wird, welche das Risiko den Materialbehälter zu kontaminieren weiter vermindert.

Die in Figur 2 dargestellte Entnahmeleitung 10 weist den Leitungsinnenraum 14 auf, in welchem das Probenmaterial transportiert wird. In der bevorzugten Ausführungsvariante der Probenahmevorrichtung 1 ist die Sterilisationseinheit mit dem Leitungsinnenraum 14 verbunden, wobei die Sterilisationseinheit den Leitungsinnenraum 14 sterilisiert. Diese Anordnung bietet den Vorteil, dass die Sterilisationseinheit genutzt wird um sowohl den Leitungsinnenraum 14 der Entnahmeleitung 10 als auch den Kammerinnenraum 5 der Entnahmekammer 3 zu sterilisieren, wenn die zweite Absperreinheit 13 geöffnet ist.
In einer alternativen Ausführungsvariante ist die Sterilisationseinheit sowohl mit dem Kammerinnenraum 5 als auch mit dem Leitungsinnenraum 14 verbunden.

In einer bevorzugten Ausführungsvariante weist die Probenahmevorrichtung 1 einen zweiten Temperatursensor 20 auf, welcher im Leitungsinnenraum 14 angeordnet ist. Dies bietet den Vorteil, dass hierdurch ermöglich wird, die Dampftemperatur im Leitungsinnenraum 14 zu bestimmen und somit zu verhindern dass diese unter einen bestimmten vorgegebenen Wert fällt. Besonders vorteilhaft ist, dass durch diese Überprüfung gewährleistet wird, dass der Sterilisationsprozess bei jeder Probenahme besonders effektiv erfolgt. Zusammen mit dem ersten Temperatursensor 19, welcher die Temperatur im Kammerinnenraum 5 bestimmt, wird eine besonders lückenlose Überprüfung der Dampftemperatur in der Probenahmevorrichtung 1 gewährleistet.

Die Entnahmeleitung 10, die erste Absperreinheit 2 und die zweite Absperreinheit 13 weisen in der bevorzugten Ausführungsvariante der Probenahmevorrichtung 1 einen Durchgangsquerschnitt von zumindest 30 mm auf. Die Kopplungseinheit 8 ist des Weiteren dazu ausgebildet ein Probengefäß 4, welches einen Öffnungsbereich mit einem Durchmesser von zumindest 30 mm aufweist anzukoppeln. Dies bietet den Vorteil, dass die Probenahmevorrichtung 1 auch für inhomogene Materialien wie beispielsweise Fruchtzubereitungen, welche Fruchtstücke beinhalten einsetzbar ist.

Wie bereits zuvor beschrieben, weist die Kopplungseinheit 8 in einer bevorzugten Ausführungsvariante Sterilisationsöffnungen 18 auf, welche mit der Sterilisationseinheit verbunden sind. Dies bietet auch in der Ausführungsvariante der Probenahmevorrichtung 1 gemäß Figur 2 den Vorteil, dass das Probengefäß 4 in jenem Bereich in welchen die Kopplungseinheit 8 eingreift im Zuge der Kopplung gezielt bedampft werden kann. Hierdurch ergibt sich der Vorteil, dass eventuell vorhandene Kontaminationen in diesem Bereich entfernt werden, und zusätzlich verhindert wird, dass Kontaminationen im Zuge der Kopplung in den Leitungsinnenraum 14 der Entnahmeleitung 10 gelangen. Die Sterilisationsöffnungen 18 können in einer alternativen Ausführungsvariante auch über den Leitungsinnenraum 14 mit der Sterilisationseinheit verbunden sein.

Des Weiteren weist die Probenahmevorrichtung 1 in einer bevorzugten Ausführungsvariante eine Unterdruckerzeugungseinheit 22 auf, welche mit dem Leitungsinnenraum 14 verbunden ist. Zur Unterstützung des Transports des Probenmaterials aus dem Materialbehälter in den Leitungsinnenraum 14 kann die Unterdruckerzeugungseinheit 22 vorgesehen werden, um einen Unterdruck im Leitungsinnenraum 14 zu erzeugen. Die Unterdruckerzeugungseinheit 22 kann beispielsweise als Vakuumpumpe ausgeführt sein. Die Unterdruckerzeugungseinheit 22 bietet den Vorteil, dass durch den erzeugten Unterdruck der Transport des Probenmaterials aus dem Materialbehälter in die Entnahmeleitung 10 besonders rasch erfolgt, und der Leitungsinnenraum 14 möglichst vollständig mit Probenmaterial ausgefüllt wird. Vorteilhafterweise liegt der durch die Unterdruckerzeugungseinheit 22 erzeugte Unterdruck nur geringfügig unter dem Druck, welcher im Materialbehälter vorliegt. Hierdurch wird gewährleistet, dass eventuell im Probenmaterial vorhandene Inhomogenitäten, wie beispielsweise Fruchtstücke in einem Joghurt, nicht zerstört werden. In einer alternativen Ausführungsvariante kann die Unterdruckerzeugungseinheit 22 aufgrund des nur geringen notwendigen Druckunterschiedes auch mit dem Kammerinnenraum 5 verbunden sein und einen Unterdruck im Kammerinnenraum 5 erzeugen, wodurch die Überführung des Probenmaterials aus dem Leitungsinnenraum 14 in das Probengefäß 4 zusätzlich unterstützt wird.

In einer weiteren bevorzugten Ausführungsvariante weist die Probenahmevorrichtung 1 eine Inertgasspüleinheit 21 auf, welche mit dem Leitungsinnenraum 14 verbunden ist. Die Inertgasspüleinheit 21 erzeugt im Leitungsinnenraum 14 einen Überdruck in Relation zum Umgebungsdruck welcher um die Probenahmevorrichtung 1 herrscht, wodurch auch das sich im Leitungsinnenraum 14 befindliche Probenmaterial unter Druck gesetzt wird. Durch diesen Überdruck wird der Transport des Probenmaterials aus der Entnahmeleitung 10 in das Probengefäß 4 unterstützt. Besonders vorteilhaft ist hierbei, dass dadurch das Risiko vermindert wird, dass bei der Probenahme Rückstände von Probenmaterial in der Entnahmeleitung 10 verbleiben. Als Inertgas kann beispielsweise Stickstoff zur Anwendung kommen. Vorteilhafterweise wird durch die Inertgasspüleinheit 21 ein Druck erzeugt, welcher nur geringfügig über dem Umgebungsdruck, welcher um die Probenahmevorrichtung 1 herrscht, liegt. Hierdurch wird analog wie bei der Unterdruckerzeugungseinheit 22 gewährleistet, dass eventuell im Probenmaterial vorhandene Inhomogenitäten, wie beispielsweise Fruchtstücke in einem Joghurt, nicht zerstört werden.

Wie in Figur 1 und Figur 2 dargestellt, weist die Entnahmekammer 3 in der bevorzugten Ausführungsvariante der Probenahmevorrichtung 1 einen in Betriebslage unterhalb der Manipulationseinheit 6 angeordneten Haltebügel 15 auf. Der Haltebügel 15 sichert das Probengefäß 4 in der Manipulationseinheit 6, wodurch der Vorteil erreicht wird, dass das Probengefäß 4 nicht aus der Öffnung 9 Manipulationseinheit 6 gleiten kann. Besonders vorteilhaft ist, dass hierdurch gewährleistet wird, dass kein Heißdampf aus dem Kammerinnenraum 5 entweichen, und keine Kontamination in den Kammerinnenraum 5 eindringen kann.

In einer besonders bevorzugten Ausführungsvariante der Probenahmevorrichtung 1 ist der Haltebügel 15 in Betriebslage unterhalb der Kopplungseinheit 8 angeordnet. Dies bietet den Vorteil, dass bei einer unbeabsichtigten Trennung der Kopplung zwischen der Kopplungseinheit 8 und dem Probengefäß 4 während der Probenahme kein Probenmaterial aus der Entnahmekammer 3 entweichen kann. Hierdurch verbleibt das Probenmaterial in einer sterilen Umgebung.

Bei einer Probename mit der Probenahmevorrichtung 1 gemäß Figur 2 weist diese folgende Betriebsabfolge auf: Zuerst wird der Leitungsinnenraum 14 und der Kammerinnenraums 5 mit der Sterilisationseinheit sterilisiert. In der bevorzugten Ausführungsvariante der Probenahmevorrichtung 1 wird hierbei der Leitungsinnenraum 14 und der Kammerinnenraum 5 bei geschlossener zweiter Absperreinheit 13 und geöffneter erster Absperreinheit 2 mit Heißdampf einer Temperatur von mindestens 120 °C geflutet, wodurch eventuell vorhandene Keime in der Probenahmevorrichtung 1 abgetötet werden. Im Anschluss wird die durch die Sterilisationseinheit erzeugte Dampfmenge reduziert, die Dampftemperatur auf unter 120 °C, vorzugsweise auf ca. 70 °C bis 80 °C, abgesenkt, und das zuvor sterilisierte Probengefäß 4 in den Kammerinnenraum 5 eingeführt. Hierdurch wird gewährleistet, dass das Bedienpersonal der Probenahmevorrichtung 1 das Probengefäß 4 manuell in den Kammerinnenraum 5 einführen kann, ohne Gefahr zu laufen Verbrühungsverletzungen durch zu hohe Dampftemperaturen zu erleiden. Im Anschluss wird das Probengefäß 4 mit der Öffnungseinheit 7 geöffnet, wobei ein Verschluss des Probengefäßes 4 an der Öffnungseinheit 7 verbleibt. Im Anschluss wird das Probengefäß 4 mit der Kopplungseinheit 8 gekoppelt, wodurch das Probengefäß 4 mit dem Materialbehälter verbunden wird. Während des Kopplungsvorgangs wird das Probengefäß 4, insbesondere in jenem Bereich in welchen die Kopplungseinheit 8 eingreift, kontinuierlich durch die Sterilisationsöffungen 18 der Kopplungseinheit 8 bedampft. Hierdurch ergibt sich der Vorteil, dass eventuell vorhandene Kontaminationen in diesem Bereich entfernt werden, und zusätzlich verhindert wird, dass eventuell mit dem Probengefäß 4 in den Kammerinnenraum 5 eingebrachte Kontaminationen im Zuge der Kopplung in den Leitungsinnenraum 14 der Entnahmeleitung 10 gelangen. Im Anschluss wird die erste Absperreinheit 2 geschlossen und die zweite Absperreinheit 13 geöffnet wodurch der Leitungsinnenraum 14 mit Probenmaterial aus dem Materialbehälter befüllt wird. In weiterer Folge wird die zweite Absperreinheit 13 wieder geschlossen und durch Öffnen der ersten Absperreinheit 2 wird das Probengefäß 4 mit dem Probenmaterial aus der Entnahmeleitung 10 befüllt. Danach wird die erste Absperreinheit 2 geschlossen, und das Probengefäß 4 mit der Öffnungseinheit 7 und dem an dieser verbliebenen Verschluss verschlossen. Zuletzt wird das Probengefäß 4 aus dem Kammerinnenraum 5 entfernt.

In einer bevorzugten Ausführungsvariante des Verfahrens wird vor der Befüllung des Leitungsinnenraums 14 mit Probenmaterial aus dem Materialbehälter ein leichter Unterdruck im Leitungsinnenraum 14 durch die Unterdruckerzeugungseinheit 22 erzeugt. Hierdurch wird der Vorteil erreicht, dass das Probenmaterial besonders rasch in die Entnahmeleitung 10 übergeführt wird, und der Leitungsinnenraum 14 möglicht vollständig mit Probenmaterial befüllt wird. Im Anschluss wird im Leitungsinnenraum 14 ein Überdruck von der Inertgasspüleinheit 21 erzeugt, wodurch das Probenmaterial nach dem Öffnen der ersten Absperreinheit 2 möglichst vollständig in das Probengefäß 4 übergeführt wird. Hierdurch wird der Vorteil erreicht, dass die Entnahmeleitung 10 möglichst rückstandsfrei entleert wird.

Das erfindungsgemäße Verfahren zur Befüllung eines Probengefäßes 4 mit Probenmaterial aus einem Materialbehälter weist folgende Verfahrensschritte auf: Zuerst wird der Kammerinnenraum 5 mit Heißdampf einer Temperatur von zumindest 120 °C geflutet. Hierbei wird der Kammerinnenraum 5 sterilisiert. Im Anschluss wird die Temperatur des Heißdampfes auf unter 120 °C reduziert. Hierdurch wird der Vorteil erreicht, dass Bedienpersonal der Probenahmevorrichtung 1 das Probengefäß 4 manuell in den Kammerinnenraum 5 einführen kann, ohne Gefahr zu laufen Verbrühungsverletzungen durch zu hohe Dampftemperaturen zu erleiden, oder zusätzliche Schutzkleidung verwenden zu müssen. Nach der Reduktion der Temperatur wird das Probengefäß 4 in den Kammerinnenraum 5 eingeführt und danach geöffnet. Im Anschluss wird das Probengefäß 4 mit dem Materialbehälter verbunden, und mit Probenmaterial aus dem Materialbehälter befüllt. Hiernach wird das Probengefäß wieder verschlossen. Vorzugsweise erfolgt das Verschließen des Probengefäßes unter Heißdampf, wodurch der Vorteil erreicht wird, dass das Risiko das Probenmaterial im Probengefäß 4 während dieses Schrittes zu kontaminieren verringert wird. Zuletzt wird das Probengefäß 4 aus dem Kammerinnenraum 5 entfernt. Das erfindungsgemäße Verfahren bietet den Vorteil, dass es eine völlig aseptische Probenahme gewährleistet, ohne die Gefahr das Probenmaterial oder den Materialbehälter zu kontaminieren.

In einer bevorzugten Ausführungsvariante des Verfahrens wird im Leitungsinnenraum 14 der Probenahmevorrichtung 1 ein Unterdruck erzeugt, und Probenmaterial in den Leitungsinnenraum 14 befördert, nachdem das Probengefäß 4 mit dem Materialbehälter verbunden wurde. Im Anschluss daran wird ein Überdruck im Leitungsinnenraum 14 erzeugt, und das Probenmaterial in das Probengefäß 4 eingefüllt. Hierdurch wird der Vorteil erreicht, dass der Leitungsinnenraum 14 möglichst vollständig mit Probenmaterial befüllt, und anschließend wieder möglichst vollständig entleert wird. Hierdurch werden nachfolgende Reinigungsschritte vereinfacht, und es wird sichergestellt, dass bei jeder Probenahme die gleiche Menge an Probenmaterial aus dem Materialbehälter entnommen wird.

## Patentansprüche

1. Probenahmevorrichtung (1) zur Befüllung eines Probengefäßes (4) mit Probenmaterial aus einem Materialbehälter, wobei die Probenahmevorrichtung (1) eine erste Absperreinheit (2), eine Sterilisationseinheit und eine Entnahmekammer (3) umfasst, wobei die Entnahmekammer (3) einen Kammerinnenraum (5), eine Manipulationseinheit (6), eine in dem Kammerinnenraum (5) angeordnete Öffnungseinheit (7) sowie eine in dem Kammerinnenraum (5) angeordnete Kopplungseinheit (8) aufweist, die erste Absperreinheit (2) zwischen dem Materialbehälter und der Kopplungseinheit (8) angeordnet ist, und die Sterilisationseinheit mit dem Kammerinnenraum (5) verbunden und dazu ausgebildet ist, den Kammerinnenraum (5) zu sterilisieren, wobei die Manipulationseinheit (6) dazu ausgebildet ist das Probengefäß (4) zumindest teilweise in den Kammerinnenraum (5), beweglich zwischen der Öffnungseinheit (7) und der Kopplungseinheit (8), aufzunehmen, die Öffnungseinheit (7) dazu ausgebildet ist das Probengefäß (4) zu öffnen und zu schließen, und die Kopplungseinheit (8) mit dem Probengefäß (4) koppelbar ausgebildet ist.

2. Probenahmevorrichtung (1) gemäß Anspruch 1, **dadurch gekennzeichnet, dass** die Kopplungseinheit (8) Sterilisationsöffnungen (18) aufweist, und die Sterilisationsöffnungen (18) mit der Sterilisationseinheit verbunden sind.

3. Probenahmevorrichtung (1) gemäß Anspruch 1 oder 2, **dadurch gekennzeichnet, dass** die Probenahmevorrichung (1) einen ersten Temperatursensor (19) aufweist, welcher im Kammerinnenraum (5) angeordnet ist.

4. Probenahmevorrichtung (1) gemäß einem der Ansprüche 1 bis 3, **dadurch gekennzeichnet, dass**
die Probenahmevorrichtung (1) eine Entnahmeleitung (10) mit einem Leitungsinnenraum (14) aufweist, welche ein erstes Ende (11), an welchem die erste Absperreinheit (2) angeordnet ist, und ein zweites Ende (12), an welchem eine zweite Absperreinheit (13) angeordnet ist aufweist, wobei das zweite Ende (12) der Entnahmeleitung (10) mit dem Materialbehälter verbunden ist, und die erste Absperreinheit (2) und die zweite Absperreinheit (13) dazu ausgebildet sind die Entnahmeleitung (10) abzusperren.

5. Probenahmevorrichtung (1) gemäß Anspruch 4, **dadurch gekennzeichnet, dass** der Leitungsinnenraum (14) ein Volumen aufweist, welches ein Volumen von Probenmaterial zur Befüllung des Probengefäßes (4) bestimmt.

6. Probenahmevorrichtung (1) gemäß Anspruch 4 oder 5, **dadurch gekennzeichnet, dass** die Sterilisationseinheit mit dem Leitungsinnenraum (14) verbunden und ausgebildet ist, den Leitungsinnenraum (14) zu sterilisieren.

7. Probenahmevorrichtung (1) gemäß einem der Ansprüche 4 bis 6, **dadurch gekennzeichnet, dass** die Probenahmevorrichung (1) einen zweiten Temperatursensor (20) aufweist, welcher im Leitungsinnenraum (14) angeordnet ist.

8. Probenahmevorrichtung (1) gemäß einem der Ansprüche 4 bis 7, **dadurch gekennzeichnet, dass** die Probenahmevorrichtung (1) eine Inertgasspüleinheit (21) aufweist, welche mit dem Leitungsinnenraum (14) verbunden und dazu ausgebildet ist, einen Überdruck im Leitungsinnenraum (14) zu erzeugen.

9. Probenahmevorrichtung (1) gemäß einem der Ansprüche 4 bis 8, **dadurch gekennzeichnet, dass** die Probenahmevorrichtung (1) eine Unterdruckerzeugungseinheit (22) aufweist, welche mit dem Leitungsinnenraum (14) verbunden, und dazu ausgebildet ist einen Unterdruck im Leitungsinnenraum (14) zu erzeugen.

10. Probenahmevorrichtung (1) gemäß einem der Ansprüche 1 bis 9, **dadurch gekennzeichnet, dass** die erste Absperreinheit (2), einen Durchgangsquerschnitt von zumindest 30 mm aufweist, und die Kopplungseinheit (8) mit einem Probengefäß (4), welches einen Öffnungsbereich mit einem Durchmesser von zumindest 30 mm aufweist, koppelbar ausgebildet ist.

11. Probenahmevorrichtung (1) gemäß einem der Ansprüche 4 bis 9, **dadurch gekennzeichnet, dass** die Entnahmeleitung (10), die erste Absperreinheit (2) und die zweite Absperreinheit (13) einen Durchgangsquerschnitt von zumindest 30 mm aufweisen, und die Kopplungseinheit (8) mit einem Probengefäß (4), welches einen Öffnungsbereich mit einem Durchmesser von zumindest 30 mm aufweist, koppelbar ausgebildet ist

12. Probenahmevorrichtung (1) gemäß einem der Ansprüche 1 bis 11, **dadurch gekennzeichnet, dass** die Entnahmekammer (3) Verbindungselemente (16) aufweist, wobei die Manipulationseinheit (6) in den Verbindungselementen (16) verschiebbar aufgenommen ist.

13. Probenahmevorrichtung (1) gemäß einem der Ansprüche 1 bis 12, **dadurch gekennzeichnet, dass** die Manipulationseinheit (6) als eine verschiebbare Begrenzungsfläche der Entnahmekammer (3) ausgebildet ist, welche eine, einem Querschnitt des Probengefäßes (4) angepasste, Öffnung (9) aufweist.

14. Probenahmevorrichtung (1) gemäß einem der Ansprüche 1 bis 13, **dadurch gekennzeichnet, dass** die Manipulationseinheit (6) ein Griffstück (17) aufweist.

15. Probenahmevorrichtung (1) gemäß einem der Ansprüche 1 bis 14, **dadurch gekennzeichnet, dass** die Entnahmekammer (3) einen in Betriebslage unterhalb der Manipulationseinheit (6) angeordneten Haltebügel (15) aufweist.

16. Probenahmevorrichtung (1) gemäß Anspruch 13 und Anspruch 15, **dadurch gekennzeichnet, dass** der Haltebügel (15) in Betriebslage unterhalb der Kopplungseinheit (8) angeordnet ist.

17. Probenahmevorrichtung (1) gemäß einem der Ansprüche 1 bis 16, **dadurch gekennzeichnet, dass** die Sterilisationseinheit ein Dampferzeuger ist.

18. Probenahmevorrichtung (1) gemäß Anspruch 17, **dadurch gekennzeichnet, dass** der Dampferzeuger Dampf einer Temperatur von zumindest 120 °C erzeugt.

19. Verfahren zur Befüllung eines Probengefäßes (4) mit Probenmaterial aus einem Materialbehälter mit einer Probenahmevorrichtung (1) gemäß einem der Ansprüche 1 bis 18 umfassend die Schritte:
a) Fluten eines Kammerinnenraums (5) der Probenahmevorrichtung (1) mit Heißdampf einer Temperatur von zumindest 120 °C;
b) Reduzieren der Temperatur des Heißdampfes auf unter 120 °C;
c) Einführen des Probengefäßes (4) in den Kammerinnenraum (5);
d) Öffnen des Probengefäßes (4);
e) Verbinden des Probengefäßes (4) mit dem Materialbehälter;
f) Befüllen des Probengefäßes (4) mit Probenmaterial aus dem Materialbehälter;
g) Schließen des Probengefäßes (4) vorzugsweise unter Heißdampf;
h) Entfernen des Probengefäßes (4) aus dem Kammerinnenraum (5).

20. Verfahren gemäß Anspruch 19, umfassend die Schritte:
i) Schließen der ersten Absperreinheit (2) und der zweiten Absperreinheit (13), gefolgt von Erzeugen eines Unterdrucks in einem Leitungsinnenraum (14) der Probenahmevorrichtung (1), und anschließend Öffnen der zweiten Absperreinheit (13) und Befördern des Probenmaterials in den Leitungsinnenraum (14) nach Schritt e);
j) Schließen der zweiten Absperreinheit (13) gefolgt von Erzeugen eines Überdrucks im Leitungsinnenraum (14) und anschließend Öffnen der ersten Absperreinheit (2) nach Schritt i) und vor Schritt f).

## Claims

1. A sampling device (1) for filling a sample vessel (4) with sample material from a material container, wherein the sampling device (1) comprises a first locking unit (2), a sterilization unit and a withdrawal chamber (3), wherein the withdrawal chamber (3) comprises an internal chamber space (5), a manipulation unit (6), an opening unit (7) arranged within the internal chamber space (5) as well as a coupling unit (8) arranged within the internal chamber space (5), the first locking unit (2) is arranged between the material container and the coupling unit (8), and the sterilization unit is connected to the internal chamber space (5) and is designed for sterilizing the internal chamber space (5), wherein the manipulation unit (6) is designed for accommodating the sample vessel (4) at least partially within the internal chamber space (5) movably between the opening unit (7) and the coupling unit (8), the opening unit (7) is designed for opening and closing the sample vessel (4), and the coupling unit (8) is designed so as to be coupleable to the sample vessel (4).

2. A sampling device (1) according to claim 1, **characterized in that** the coupling unit (8) has sterilization openings (18) and the sterilization openings (18) are connected to the sterilization unit.

3. A sampling device (1) according to claim 1 or 2, **characterized in that** the sampling device (1) has a first temperature sensor (19) arranged within the internal chamber space (5).

4. A sampling device (1) according to any of claims 1 to 3, **characterized in that** the sampling device (1) has a withdrawal line (10) comprising an internal line space (14), the withdrawal line having a first end (11) at which the first locking unit (2) is arranged and a second end (12) at which a second locking unit (13) is arranged, with the second end (12) of the withdrawal line (10) being connected to the material container and the first locking unit (2) and the second locking unit (13) being designed for shutting off the withdrawal line (10).

5. A sampling device (1) according to claim 4, **characterized in that** the internal line space (14) has a volume which determines a volume of sample material for filling the sample vessel (4).

6. A sampling device (1) according to claim 4 or 5, **characterized in that** the sterilization unit is connected to the internal line space (14) and is designed for sterilizing the internal line space (14).

7. A sampling device (1) according to any of claims 4 to 6, **characterized in that** the sampling device (1) has a second temperature sensor (20) arranged within the internal line space (14).

8. A sampling device (1) according to any of claims 4 to 7, **characterized in that** the sampling device (1) has an inert-gas purging unit (21) which is connected to the internal line space (14) and is designed for generating a positive pressure within the internal line space (14).

9. A sampling device (1) according to any of claims 4 to 8, **characterized in that** the sampling device (1) has a negative pressure generating unit (22) which is connected to the internal line space (14) and is designed for generating a negative pressure within the internal line space (14).

10. A sampling device (1) according to any of claims 1 to 9, **characterized in that** the first locking unit (2) has a passage cross-section of at least 30 mm and the coupling unit (8) is designed so as to be coupleable to a sample vessel (4), which has an opening area with a diameter of at least 30 mm.

11. A sampling device (1) according to any of claims 4 to 9, **characterized in that** the withdrawal line (10), the first locking unit (2) and the second locking unit (13) have a passage cross-section of at least 30 mm and the coupling unit (8) is designed so as to be coupleable to a sample vessel (4), which has an opening area with a diameter of at least 30 mm.

12. A sampling device (1) according to any of claims 1 to 11, **characterized in that** the withdrawal chamber (3) has connecting elements (16), with the manipulation unit (6) being accommodated slidably within the connecting elements (16).

13. A sampling device (1) according to any of claims 1 to 12, **characterized in that** the manipulation unit (6) is designed as a slidable boundary surface of the withdrawal chamber (3), which has an opening (9) adapted to a cross-section of the sample vessel (4).

14. A sampling device (1) according to any of claims 1 to 13, **characterized in that** the manipulation unit (6) has a handle piece (17).

15. A sampling device (1) according to any of claims 1 to 14, **characterized in that** the withdrawal chamber (3) has a retaining bracket (15), which, in the operating position, is arranged underneath the manipulation unit (6).

16. A sampling device (1) according to claim 13 and claim 15, **characterized in that** the retaining bracket (15) is arranged, in the operating position, underneath the coupling unit (8).

17. A sampling device (1) according to any of claims 1 to 16, **characterized in that** the sterilization unit is a steam generator.

18. A sampling device (1) according to claim 17, **characterized in that** the steam generator generates steam of a temperature of at least 120°C.

19. A method of filling a sample vessel (4) with sample material from a material container using a sampling device (1) according to any of claims 1 to 18, comprising the steps of:
a) flooding an internal chamber space (5) of the sampling device (1) with superheated steam of a temperature of at least 120°C;
b) reducing the temperature of the superheated steam to below 120°C;
c) introducing the sample vessel (4) into the internal chamber space (5);
d) opening the sample vessel (4);
e) connecting the sample vessel (4) to the material container;
f) filling the sample vessel (4) with sample material from the material container;
g) closing the sample vessel (4) preferably under superheated steam;
h) removing the sample vessel (4) from the internal chamber space (5).

20. A method according to claim 19, comprising the steps of:
i) closing the first locking unit (2) and the second locking unit (13), followed by generating a negative pressure in an internal line space (14) of the sampling device (1) and, subsequently, opening the second locking unit (13) and conveying the sample material into the internal line space (14) after step e);
j) closing the second locking unit (13), followed by generating a positive pressure in the internal line space (14) and, subsequently, opening the first locking unit (2) after step i) and before step f).

## Revendications

1. Dispositif d'échantillonnage (1) pour remplir un récipient d'échantillon (4) avec un matériau d'échantillon provenant d'un récipient de matériau, le dispositif d'échantillonnage (1) comprenant une première unité de verrouillage (2), une unité de stérilisation et une chambre de prélèvement (3), où la chambre de prélèvement (3) comporte un espace intérieur de la chambre (5), une unité de manipulation (6), une unité d'ouverture (7) disposée dans l'espace intérieur de la chambre (5) ainsi qu'une unité d'accouplement (8) disposée dans l'espace intérieur de la chambre (5), où la première unité de verrouillage (2) est disposée entre le récipient de matériau et l'unité d'accouplement (8) est reliée à l'espace intérieur de la chambre (5) et est conçue de manière à stériliser l'espace intérieur de la chambre (5), l'unité de manipulation (6) étant conçue pour recevoir le récipient d'échantillon (4), au moins partiellement dans l'espace intérieur de la chambre (5), qui est mobile entre l'unité d'ouverture (7) et l'unité d'accouplement (8), et l'unité d'ouverture (7) étant conçue pour ouvrir et fermer le récipient d'échantillon (4) et où l'unité d'accouplement (8) est conçue pour être accouplée au récipient d'échantillon (4).

2. Dispositif d'échantillonnage (1) selon la revendication 1, **caractérisé en ce que** l'unité d'accouplement (8) présente des ouvertures de stérilisation (18) et que les ouvertures de stérilisation (18) sont reliées à l'unité de stérilisation.

3. Dispositif d'échantillonnage (1) selon la revendication 1 ou 2, **caractérisé en ce que** le dispositif d'échantillonnage (1) comporte un premier capteur de température (19) qui est disposé dans l'espace intérieur de la chambre (5).

4. Dispositif d'échantillonnage (1) selon l'une des revendications 1 à 3, **caractérisé en ce que** le dispositif d'échantillonnage (1) présente une conduite de prélèvement (10) avec un espace intérieur de conduite (14) qui présente une première extrémité (11), sur laquelle est disposée la première unité de verrouillage (2), et une seconde extrémité (12), sur laquelle est disposée une deuxième unité de verrouillage (13), la deuxième extrémité (12) de la conduite de prélèvement (10) étant reliée au récipient de matériau et la première unité de verrouillage (2) et la deuxième unité de verrouillage (13) étant conçues pour verrouiller la conduite d'extraction (10).

5. Dispositif d'échantillonnage (1) selon la revendication 4, **caractérisé en ce que** l'espace intérieur de la conduite (14) présente un volume qui détermine un volume de matériau d'échantillonnage pour remplir le récipient d'échantillonnage (4).

6. Dispositif d'échantillonnage (1) selon la revendication 4 ou 5, **caractérisé en ce que** l'unité de stérilisation est reliée dans l'espace intérieur (14) de la conduite et qu'elle est conçue pour stériliser l'espace intérieur (14) de la conduite.

7. Dispositif d'échantillonnage (1) selon l'une des revendications 4 à 6, **caractérisé en ce que** le dispositif d'échantillonnage (1) comporte un deuxième capteur de température (20) qui est disposé dans l'espace intérieur de la conduite (14).

8. Dispositif d'échantillonnage (1) selon l'une des revendications 4 à 7, **caractérisé en ce que** le dispositif d'échantillonnage (1) comprend une unité de rinçage au gaz inerte (21) reliée à l'espace intérieur (14) de la conduite et adaptée pour générer une surpression dans l'espace intérieur (14) de la conduite.

9. Dispositif d'échantillonnage (1) selon l'une des revendications 4 à 8, **caractérisé en ce que** le dispositif d'échantillonnage (1) comprend une unité génératrice de vide (22) reliée dans l'espace intérieur de la conduite (14) et adaptée pour générer un vide dans l'espace intérieur de la conduite (14).

10. Dispositif d'échantillonnage (1) selon l'une des revendications 1 à 9, **caractérisé en ce que** la première unité de verrouillage (2) présente une section transversale de passage d'au moins 30 mm et que l'unité d'accouplement (8) est conçue de manière à pouvoir être couplée à un récipient d'échantillon (4) qui présente une zone d'ouverture d'un diamètre d'au moins 30 mm.

11. Dispositif d'échantillonnage (1) selon l'une des revendications 4 à 9, **caractérisé en ce que** la conduite de prélèvement (10), la première unité de verrouillage (2) et la deuxième unité de verrouillage (13) ont une section transversale de passage d'au moins 30 mm et que l'unité d'accouplement (8) est conçue de manière à pouvoir être couplée à un récipient d'échantillon (4) qui présente une zone d'ouverture d'un diamètre d'au moins 30 mm.

12. Dispositif d'échantillonnage (1) selon l'une des revendications 1 à 11, **caractérisé en ce que** la chambre de prélèvement (3) présente des éléments de liaison (16), l'unité de manipulation (6) étant logée de manière mobile dans les éléments de liaison (16).

13. Dispositif d'échantillonnage (1) selon l'une des revendications 1 à 12, **caractérisé en ce que** l'unité de manipulation (6) est réalisée sous la forme d'une surface limite mobile de la chambre de prélèvement (3), qui présente une ouverture (9) adaptée à une section transversale du récipient d'échantillon (4).

14. Dispositif d'échantillonnage (1) selon l'une des revendications 1 à 13, **caractérisé en ce que** l'unité de manipulation (6) présente une poignée (17).

15. Dispositif d'échantillonnage (1) selon l'une des revendications 1 à 14, **caractérisé en ce que** la chambre de prélèvement (3) présente un étrier de retenue (15) disposé en position de fonctionnement sous l'unité de manipulation (6).

16. Dispositif d'échantillonnage (1) selon les revendications 13 et 15, **caractérisé en ce que** l'étrier de retenue (15) est disposé en position de fonctionnement sous l'unité d'accouplement (8).

17. Dispositif d'échantillonnage (1) selon l'une quelconque des revendications 1 à 16, **caractérisé en ce que** l'unité de stérilisation est un générateur de vapeur.

18. Dispositif d'échantillonnage (1) selon la revendication 17, **caractérisé en ce que** le générateur de vapeur produit de la vapeur à une température d'au moins 120°C.

19. Procédé de remplissage d'un récipient d'échantillon (4) avec un matériau d'échantillon provenant d'un récipient de matériau avec un dispositif d'échantillonnage (1) selon l'une quelconque des revendications 1 à 18, comprenant les étapes consistant à :
a) inonder un espace intérieur de la chambre (5) du dispositif d'échantillonnage (1) par de la vapeur surchauffée à une température d'au moins 120°C ;
b) baisser la température de la vapeur surchauffée en dessous de 120 °C ;
c) introduire le récipient d'échantillon (4) dans l'espace intérieur de la chambre (5) ;
d) ouvrir le récipient d'échantillon (4) ;
e) relier le récipient d'échantillon (4) au récipient de matériau ;
f) remplir le récipient d'échantillon (4) avec le matériau d'échantillon provenant du récipient de matériau ;
g) fermer le récipient d'échantillon (4), de préférence avec de la vapeur surchauffée ;
h) extraire le récipient d'échantillon (4) de l'espace intérieur de la chambre (5).

20. Procédé selon la revendication 19 comprenant les étapes suivantes consistant à :
i) fermer la première unité de verrouillage (2) et de la deuxième unité de verrouillage (13), puis générer un vide dans un espace intérieur de la conduite (14) du dispositif d'échantillonnage (1), ensuite ouvrir la deuxième unité de verrouillage (13) et transporter le matériau échantillon dans l'espace intérieur de conduite (14) après l'étape e) ;
j) fermer la deuxième unité de verrouillage (13), puis générer une surpression à l'espace intérieur de la conduite (14) et ouvrir la première unité de verrouillage (2) après l'étape i) et avant l'étape f).
